# EUROPEAN PATENT SPECIFICATION

(11) **EP 2 254 463 B1**
(45) Date of publication and mention of the grant of the patent: **27.05.2020**
(21) Application number: 09727472.4
(22) Date of filing: 02.04.2009
(51) Int. Cl.: A61B 5/00, A61B 1/06, A61B 1/00

(54) **PHOTODYNAMIC-BASED MYOCARDIAL MAPPING DEVICE**
PHOTODYNAMIKBASIERTE MYOKARDIALE ABBILDUNGSVORRICHTUNG
DISPOSITIF DE MISE EN CORRESPONDANCE MYOCARDIQUE À BASE PHOTODYNAMIQUE

(30) Priority: 02.04.2008 US 41713 P
(43) Date of publication of application: 01.12.2010
(73) Proprietor: St. Jude Medical, Atrial Fibrillation Division, Inc., St. Paul, Minnesota 55117-9913 (US)
(72) Inventor: PAUL, Saurav, Minneapolis, MN 55414 (US); JUST, Dale, Minneapolis MN 55407 (US); SKOGLUND, Jamie, Plymouth MN 55447 (US); BYRD, Israel, Alexander, Richfield MN 55423 (US); MCDOWALL, Paul, Eden Prairie MN 55346 (US)
(74) Representative: Kramer Barske Schmidtchen Patentanwälte PartG mbB
(86) International application number: PCT/US2009/039367
(87) International publication number: WO 2009/124220

(56) References cited:
- EP-A1- 1 870 131
- WO-A1-96/13205
- US-A- 5 219 527
- US-A- 5 239 998
- US-A- 5 678 550
- US-A1- 2004 260 182
- US-A1- 2007 208 257
- US-A1- 2008 045 842
- US-B1- 6 811 562
- US-B1- 7 344 528

## Description

### BACKGROUND OF THE INVENTION

### a. Field of the Invention

The invention relates to a photodynamic-based myocardial mapping device and methods using the same for mapping and therapy, including delivery of therapeutic agents. More particularly, the invention relates to a fiber optic based catheter and method for optical myocardial mapping and photodynamic-based myocardial therapy.

### b. Background Art

US 2004/0260182 A1 discloses an intraluminal spectroscope with a wall contacting probe. US 5,678,550 A discloses an apparatus and method for in situ detection of areas of cardiac electrical activity. US 5,239,998 A discloses an apparatus and method for fluorescent excitation and detection from potentiometric dyes with a single-ended optical fiber.
Catheters are widely used to perform a variety of functions relating to therapeutic and diagnostic medical procedures involving tissues within a body. Typically, catheters can be inserted within a vessel located near the surface of a body (e.g., in an artery or vein in the leg, neck, or arm) and maneuvered to a region of interest within the body to enable diagnosis and treatment of tissue without the need for more invasive procedures. For example, catheters can be inserted into a body during mapping, ablation, and/or therapy delivery procedures performed on tissues within a body. Mapping uses a catheter with sensing electrodes to monitor various forms of electrical activity in the body. Tissue ablation can be accomplished using a catheter to apply localized energy to a selected location within the body to kill tissue. Ablation procedures can be used to treat conditions, such as atrial arrhythmia. Arrhythmia can create a variety of dangerous conditions including irregular heart rates, loss of synchronous atrioventricular contractions, and stasis of blood flow; these can lead to a variety of ailments and death. A primary cause of atrial arrhythmia involves stray electrical signals within the left or right atrium of the heart.

Optical mapping is used to explore complex cardiac electrical signal propagation. High-resolution optical mapping with voltage-sensitive dyes is used to depict complex propagation patterns of cardiac transmembrane potentials. The optical signal obtained from the tissue surface is typically the response of the transmembrane potential averaged upon depth rather than surface only. Optical mapping methods for recording biological fluorescence as a surrogate for direct transmembrane electrical measurements has also been accomplished. For example, a thorough review of the history, principles, and use of optical mapping, with particular respect to cardiac research, is disclosed in Optical Mapping of Cardiac Excitation and Arrhythmias (Futura Publishing, 2001; David S. Rosenbaum and Jose Jalife, ed.). Optical mapping techniques use imaging devices, such as photodiode arrays (PDA) or charge coupled device (CCD) cameras, with the heart tissue being illuminated and either continuously or spatially scanned. In electrically-active biological tissue, the intrinsic light scattering or birefringence signals follow the time course of the action potential signal. Alternatively, optical signals that follow the time course of the action potential can be artificially generated by infusion of drugs that are absorbed into cells or bind cell membranes that are electrically sensitive.

Known optical mapping techniques have been conducted on excised hearts perfused with crystaloid solutions rather than with blood. This is necessary to obtain a superior fidelity signal from either the inherent fluorescence or that created with detectable substances. However, recent techniques have reduced the detrimental effects of blood on fluorescence signals. The detectable substances typically used with this technique include voltage-sensitive dyes, such as the aminonaphthylethenylpyridinium (ANEP) dyes. The detectable substances that artificially produce optical signals are relatively inert until activated by light of a specific wavelength, with each drug sensitive to a particular wavelength. Upon activation, the detectable substance emits or reflects light at a different wavelength than that at which it is activated. The emitted spectrum shifts in proportion to the change of voltage across the cell membrane. For example, the voltage-sensitive dye, di-4-ANNEPS, exhibits a green shift as the intracellular voltage is increased with respect to the extracellular voltage, with a change in fluorescence of roughly 10% per 100 mV (millivolts). Di-4-ANNEPS requires excitation signals at wavelengths shorter than those in the red spectrum and also emits signals in or below the red spectrum. This produces a high signal to noise ratio in the cardiac cell, which typically shifts from -80 mV resting potential to 20 mV maximal depolarization during the action potential that precedes mechanical contraction. There are numerous other detectable substances with similar behavior but operating in different spectra or time course of response. For these cases, blood is an insurmountable source of noise. Recently, dyes that excite in the red spectrum and fluoresce in the near-infrared spectrum have been developed, and their feasibility tested (A. Matiukas et al., 2006. New near-infrared optical probes of cardiac electrical activity. Am J Physiol Heart Circ Physiol 290:H2633-H2643). Since blood should not significantly distort the excitation or emitted signal, recordings in blood perfused hearts, and therefore intact animals, are feasible.

Endoscopic fluorescence mapping of the endocardial surface has been shown in excised sheep hearts (Kalifa, et al. 2007. Endoscopic fluorescence mapping of the left atrium: A novel experimental approach for high resolution endocardial mapping in the intact heart. Heart Rhythm 4:916-924). Kalifa et al. used direct view and side view dual channel endoscopes coupled to a laser for illumination and a CCD camera for imaging and examined left atrial locations to record electrical wave propagation. They concluded that in isolated hearts, comprehensive evaluation of atrial fibrillation activity in the posterior left atrium and the efficacy of pharmacologic and ablative interventions could be accomplished. However, Kalifa et al.'s optical mapping is limited to excised hearts.

Photodynamic therapy involves using photodynamic agents, along with a light source, such as a laser, to destroy cancer cells or deliver other therapy to a target tissue. The drugs only work after they have been activated by certain wavelengths of light. Known methods and procedures exist for photodynamic ablation in ablating cancer tumors. However, photodynamic ablation applications specific to cardiac tissue do not appear to be known, other than as collateral damage after photodynamic ablation of an esophageal cancer, such as disclosed by BF Overholt et al. (1997. Photodynamic Therapy for Barrett's Esophagus: Cardiac Effects. Lasers in Surgery and Medicine, 21(4):317-20).

### BRIEF SUMMARY OF THE INVENTION

The invention includes photodynamic-based myocardial mapping devices that can be used to optically map the heart. The devices, which can be in the form of fiber optic-based catheters, can be used, among other things, to deliver photodynamic therapeutic agents that are activated upon exposure to specific light wavelengths.

The advantages of the devices and methods of the invention are manifold. For mapping functions, the procedure is free of many of the artifacts found in other mapping procedures that depend on using electrical measurements. Even touching the wall of a heart chamber can cause noise, as well as the device itself. In optical applications, such artifacts are substantially mitigated and potentially eliminated. The entire electrical cycle of cardiac activity can potentially be measured without gaps, enabling better mapping and ultimately, improved diagnosis of conditions. The devices can also be modified to provide high resolution mapping, thus pinpointing the location of aberrant electrical activity.

In therapeutic applications, the devices and methods can, for example, provide for targeted delivery of photodynamic therapeutic agents. Because the devices can provide high resolution, the target areas for therapeutic intervention can be minimized. This aspect can be accomplished in part because the catheters can be constructed at the catheter level to project the therapeutic light on one side of the device, and/or be controlled at the level of individual optical fibers. The devices and methods can also be used for cell ablation techniques, such treatment of cardiac arrhythmias.

Through various configurations, the devices and methods can be used in connection with epicardial and endocardial applications, and can be used for "whole chamber," local (conventional), and regional endocardial applications.

In a first aspect, the invention is directed to a photodynamic tissue mapping device, comprising: a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor.

The device can include a plurality of optical fibers and a plurality of optical electrodes, the plurality of optical electrodes distributed along the shaft. Optical electrodes can be, for example, point electrodes, ring electrodes, and tip electrodes. The outer surface of ring electrodes can be convex, planar, or concave. The optical electrodes can comprise glass, a solid comprising polymers, a low durometer polymer, or be encapsulated with a fluid or gel. At least one optical electrode can enable, for example, optical filtering, optical polarization or variable refractive index.

The device can have an optical filter coupled to at least one optical fiber; furthermore, the device can further comprise a light guide adapter that couples the at least one optical fiber to the at least one optical electrode. An example of a suitable light source includes at least one light emitting diode (LED); optical sensors can include a photodiode array (PDA) or charge coupled device (CCD) camera.

The device can be in the form of a catheter, such as a basket assembly, array, and non-contact mapping catheter. In the case wherein the catheter is a basket assembly catheter, it can further comprise: at least one spline having a least one opening, the at least one spline attached to a distal end of a catheter shaft; and a handle portion, wherein the at least one optical fiber is connected to, or threaded through the at least one opening in the at least one spline. Such a device can comprises a plurality of splines, the plurality of splines comprising 8 splines or 16 splines, or more. Each spline can comprise, for example, at least one opening, or 2, 3, 4, 5, 6,7, 8, 9, 10, 15, 20, 25, 30 or more openings. The end of the at least one optical fiber can be flush with a surface of the at least one spline. The device can further comprise at least one outer lumen or at least one inner lumen, wherein the plurality of optical fibers is threaded through the at least one outer or at least one inner lumen, and may further comprise at least one pull wire connected at a distal end of the device and coupled to an actuator element at a posterior end of the device.

In another aspect, methods of detecting electrical activity in a tissue target portion are disclosed, comprising providing a device that comprises: a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor; delivering to the target portion a voltage-sensitive detectable substance; positioning the distal portion of the device adjacent to the target portion; and detecting the voltage-sensitive detectable substance, wherein detecting the voltage-sensitive detectable substance detects electrical activity in the target portion. The voltage-sensitive detectable substance can be a aminonaphthylethenylpyridinium dye, which is excited with a suitable wavelength of light, which allows for detecting emitted signals from the excited aminonaphthylethenylpyridinium dye.

In another aspect, methods of delivering therapy to a target portion of a heart are disclosed, comprising providing a device, comprising: a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor; administering to the target portion of the heart a photodynamic therapeutic substance; positioning the device adjacent to the target portion of the heart; activating the photodynamic therapeutic substance by exposing the photodynamic therapeutic substance to an effective amount of light from the device; wherein activating the photodynamic therapeutic substance is delivering therapy to the target portion of the heart. For example, the photodynamic therapeutic substance, when activated, can incur necrosis or apoptosis in the target portion of the heart. The amount of the photodynamic therapeutic substance delivered to the target portion of the heart can be in some cases controlled by controlling the intensity and duration of the effective amount of light.

In another aspect, methods of optically mapping electrical activity in a target portion of a heart are disclosed, comprising providing a device, comprising: a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor; delivering to the target portion of the heart a voltage sensitive detectable substance; positioning the distal portion of the device adjacent to the target portion of the heart; and detecting the voltage sensitive detectable substance, wherein detecting the voltage sensitive detectable substance is mapping the electrical activity in the target portion of the heart.

In another aspect, methods of acquiring fluorescence signals at a rapid rate to distinguish key features of each action potential are disclosed, comprising using a fiber optic-based catheter comprising a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor.

In another aspect, methods of acquiring fluorescence signals over a long exposure time to detect depressed voltage amplitudes are disclosed, comprising using a fiber optic-based catheter comprising a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor.

In yet another aspect, methods of delivering light for photodynamic-based therapy to a target tissue are disclosed, comprising providing a device comprising: a shaft with a proximal end and a distal end; at least one optical electrode at the distal end of the shaft; at least one optical fiber positioned inside the shaft, wherein the at least one optical fiber extends from the distal end of the shaft, coupled to the at least one optical electrode at an outer surface of the shaft, to the proximal end of the shaft; a light source coupled to the at least one optical fiber at a proximal end of the at least one optical fiber; an optical sensor coupled to an optical fiber; and an analyzer coupled to the optical sensor; administering a photodynamic drug to the target tissue; activating the photodynamic drug by exposing the drug to an excitation wavelength, administered from the device.

### BRIEF DESCRIPTION OF THE DRAWINGS

Aspects, features, details, utilities, and advantages of the present invention will be apparent from reading the following description and from reviewing the accompanying drawings, wherein:
Fig. 1 is a fragmentary view in partial cross-section of a catheter.
Fig. 2 is a cross-sectional view, taken along line 2-2 of Fig. 1.
Fig. 3 is a fragmentary view of the catheter assembly of Fig. 1.
Fig. 4A is a partial side view of an embodiment of a distal end of an optical fiber-based catheter.
Fig. 4B is a cross-sectional view, taken along line 3-3 of Fig. 4A.
Fig. 5A is a partial side view of another embodiment of a distal end of an optical fiber-based catheter, the figure generally illustrating different forms of optical electrodes.
Fig. 5B is a cross-sectional view of an embodiment of a distal end of an optical fiber-based catheter, including wedge-shaped optical electrodes.
Fig. 5C is a cross-sectional view of an embodiment of a distal end of an optical fiber-based catheter, including an embodiment of wedge-shaped optical electrodes.
Fig. 6 is a partial side view of a distal end of an embodiment of an optical fiber-based catheter that includes optical electrodes generally provided in a staggered or an offset pattern or configuration.
Fig. 7 is a partial side view of a distal end of an embodiment of an optical-based catheter 10 including ring optical electrodes and a tip optical electrode.
Fig. 8A shows a partial longitudinal view of a distal end of an embodiment of an optical-based catheter with an embodiment of a tip electrode.
Fig. 8B is a fragmentary view in a longitudinal section of a distal end of an embodiment of an optical-based catheter with an embodiment of a tip electrode.
Fig. 9A is a fragmentary view in a longitudinal section of an embodiment of a distal end of an optical fiber-based catheter, showing an embodiment of a ring optical electrode having a flat surface around the circumference.
Fig. 9B is a fragmentary view in a longitudinal section of an embodiment of a distal end of an optical fiber-based catheter, showing an embodiment of a ring optical electrode having a concave surface around the circumference.
Fig. 9C is a fragmentary view in a longitudinal section of an embodiment of a distal end of an optical fiber-based catheter, showing an embodiment of a ring optical electrode having a convex surface around the circumference.
Fig. 9D is a fragmentary view in a longitudinal section of an embodiment of a distal end of an optical fiber-based catheter, showing an embodiment of a ring optical electrode having a conical shape and showing a bore to allow passage of optical fibers.
Fig. 9E is a cross-sectional view, taken along line 9E-9E of Fig. 7, showing an embodiment of a ring optical electrode having a cylindrical shape and a lumen.
Fig. 10A shows a representation of an embodiment of an optical fiber-based catheter in use in tissue provided with a voltage-sensitive detectable substance.
Fig. 10B shows a representation that generally depicts the flow of light and signals associated with an embodiment of an optical fiber-based catheter in tissue provided with a voltage-sensitive detectable substance.
Fig. 11A depicts a side view of an embodiment of a fiber optic-based catheter in an unexpanded position with a diode array.
Fig. 11B depicts a side view of an embodiment of a fiber optic-based catheter in an unexpanded position with a diode array.
Fig. 12 generally illustrates an embodiment of a fiber optic-based catheter with splines positioned about a dime coin.
Fig. 13 is a side view of an embodiment of a fiber optic-based catheter generally illustrating splines in an expanded position.
Fig. 14 is a side view of the catheter of Fig. 13, generally illustrating splines in an unexpanded position.
Fig. 15 is a side view of an embodiment of a fiber optic-based catheter, generally illustrating a distal basket assembly end and a proximal handle end.
Fig. 16 shows a side view of an embodiment of a fiber optic-based catheter showing light conduction of the basket assembly in an unexpanded position.
Fig. 17 shows a side view of an embodiment of a fiber optic-based catheter showing the basket assembly in an expanded position.
Fig. 18 shows a side view of an embodiment of a fiber optic-based catheter showing light conduction of the basket assembly in an expanded position.
Fig. 19 shows surface view of an embodiment of a spline of an embodiment of a fiber optic-based catheter.
Fig. 20 shows optical fibers inserted into holes in a spline of an embodiment of a fiber optic-based catheter.
Fig. 21 shows cut optical fibers in a spline of an embodiment of a fiber optic-based catheter.
Fig. 22 shows a side view an assembly of optical fibers and splines of an embodiment of a fiber optic-based catheter.
Fig. 23 shows a side view of an embodiment of a fiber optic-based catheter showing an outer lumen.
Fig. 24 shows a side view of an embodiment of a fiber optic-based catheter showing pull wires.
Fig. 25 shows a side view of taped optical fibers of an embodiment of a fiber optic-based catheter.
Fig. 26 shows a side view of an embodiment of a fiber optic-based catheter showing pull wires in the distal tip.

### DETAILED DESCRIPTION OF EMBODIMENTS OF THE INVENTION

Initially, various components and embodiments of fiber optic-based catheters are presented. Some examples of methods for using devices various embodiments of devices provided in accordance with embodiments of the invention are then generally disclosed.

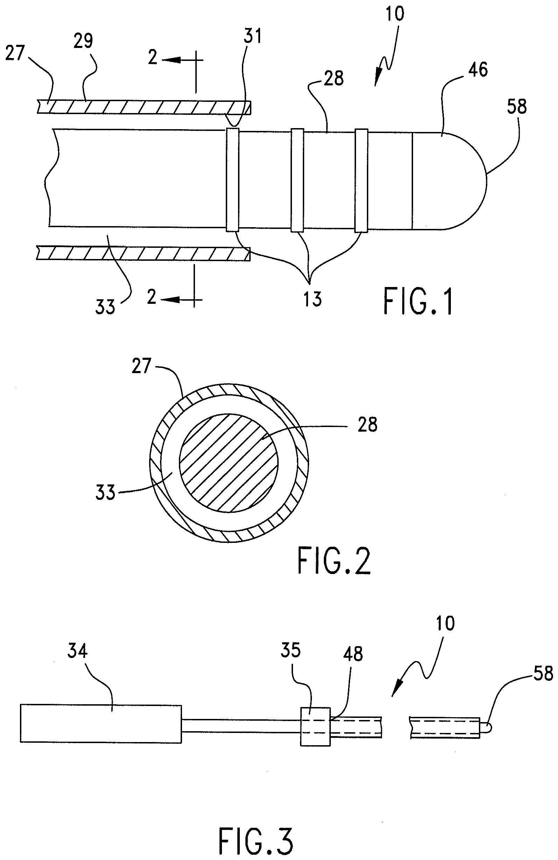

Fig. 1 is a fragmentary view in partial cross-section of a catheter assembly 10 that can be used for medical procedures, such as myocardial mapping. The catheter assembly 10 includes a catheter tip 58 at a distal end 46, a catheter shaft 28 having a plurality of electrode rings 13 around the circumference of the shaft distal portion 16, a sheath 27 having an outer surface 29 and an inner surface 31, and a lumen 33. Typically, the catheter shaft is advanced through the sheath.

Fig. 2 is a cross-sectional view, taken along line 2-2 of Fig. 1, illustrating the catheter shaft 28, sheath 27, and lumen 33. The outer diameter and inner diameter of the sheath 27 are both larger than the outer diameters of the shaft 28 and the tip 58.

Fig. 3 is a fragmentary view of the catheter assembly 10 of Fig. 1. The catheter assembly includes a proximal end 48 with a handle portion 35 and a connector member 34. The connector member 34 may comprise a standard grounding pad, quick connect, spring-loaded contact, clamp connect, or another type of electrical connector suitable for use with catheters.

The device includes catheters having proximal and distal portions. The distal portion can include a flexible region that can be flexed during deployment through a sheath as well as during placement against a heart wall. The distal portion of the catheter shaft can include a segment that is linear, preformed curvilinear, or preformed curved. The distal portion of the catheter shaft can include multiple finger-like extensions. The shaft parts (e.g., distal portion, proximal portion, and/or extensions) can be integrated or assembled from separate parts.

The catheter shaft may be a hollow shaft with an outer surface and an inner surface. The catheter shaft may have one or more lumens on its outer surface (outer lumens) and/or inner surface (inner lumens). One of ordinary skill in the art will recognize and appreciate that the catheter shaft can have one or more outer and/or inner lumens that can be interconnected to create a continuous channel and/or can have one or more lumens of various differing lengths, shapes, and distributions. In some embodiments, these lumens can be configured or used, at least in part, as irrigation channels to deliver various substances to target tissues in a selective manner.

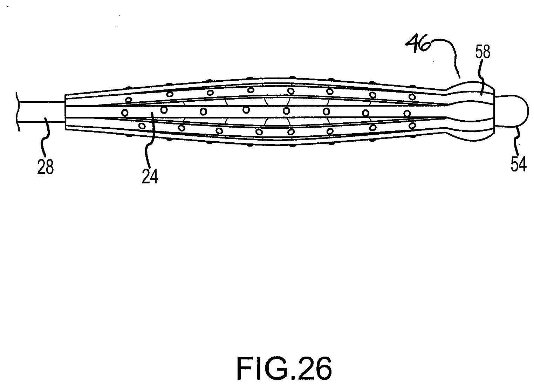

The catheter can be made steerable by the application of pull wires at the distal end of the catheter. These pull wires can be connected to an actuator element that is coupled to the catheter. A handle can be adapted to connect to the actuator element or elements so that a user can selectively manipulate the distal end of the catheter assembly to deflect in one or more directions (e.g., up, down, left, and right). The handle is operative to effect movement (i.e., deflection) of the distal end of the catheter assembly. The catheter can be modified so that a portion of the lumen extends or remains beyond the handle. The optical fibers can be inserted through the lumen from the proximal end to the distal end. The pull wires can be inserted through the inner diameter of the proximal end and the distal end of a basket assembly. Fig. 26 generally illustrates an embodiment of a fiber optic-based steerable catheter; however, various other embodiments of catheters can be outfitted similarly with pull wires.

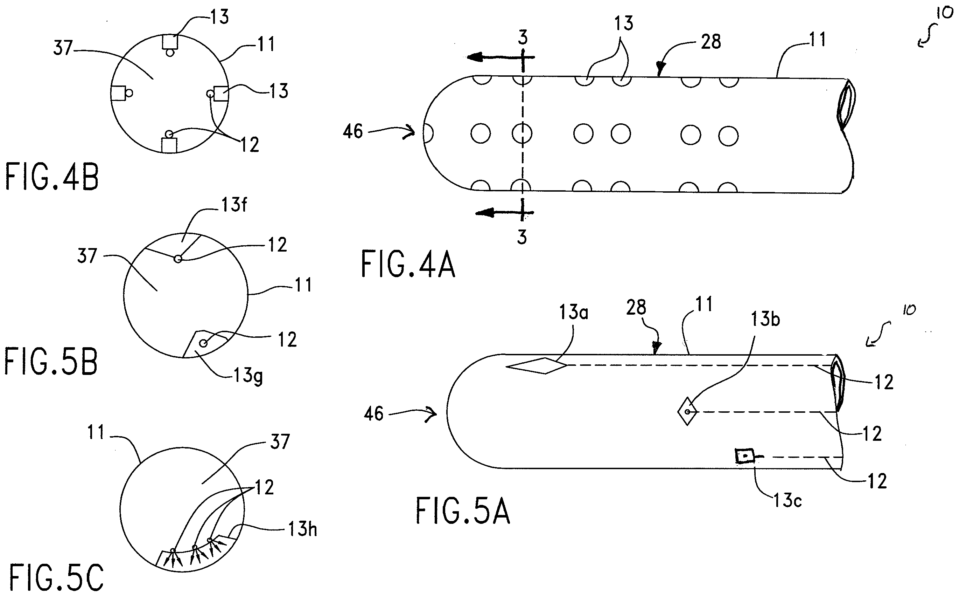

In some embodiments, optical fibers are coupled to optical electrodes provided at or about the distal end of the catheter shaft. Optical electrodes associated with embodiments of the invention can, to some extent, function in the nature of electrical electrodes in, for example, atrial fibrillation ablation catheters. However, fiber optic-based catheters can measure the optical response of a voltage-sensitive dye (an electrically responsive chemical) that changes its color in response to changes in transmembrane potential (an electrical characteristic of the tissue). As such, the optical electrodes can be considered to optically "contact" the electrical circuit of the tissue. As generally illustrated in Figs. 4B, 5A, 6 and 7, for example, optical electrodes 13 can be embedded in, or attached to, the surface of the catheter wall 11 at or about the distal portion 46 of the catheter shaft 28. The optical electrodes 13 can be configured to transmit light from the optical fibers 12 to a target tissue, as well as function to aid in sensing light by transmitting emitted light from the target tissue to an optical fiber 12.

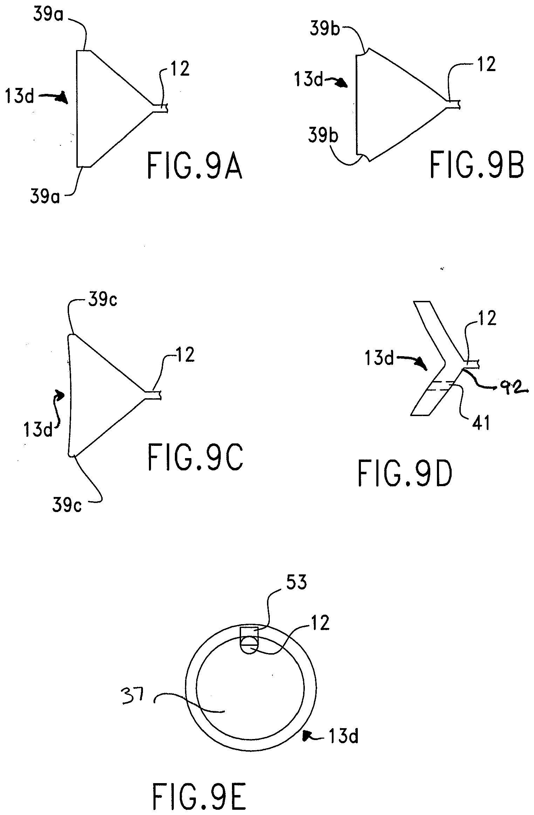

As further generally shown in Figs. 4B, 5A-C, 6, 8A, 8B, and 9A-9E, the optical electrodes 13 are coupled to the optical fibers 12. Optical electrodes 13 can be configured in various shapes, such as, without limitation, wedge, square, diamond, or circular shapes. Further, optical electrodes, can be configured as point, ring, or tip optical electrodes. Moreover, for some embodiments, including those illustrated in Fig. 9, the external surface of optical electrodes 13, which can be at or about the surface of the catheter wall 11, can be generally flat, concave or convex.

Optical electrodes can be solid and manufactured using glass or polymers. In other embodiments, optical electrodes can also be compliant, such as in the case of flexible tip catheters, in which case they are manufactured using low durometer polymers and can be encapsulated in suitable fluids or gels.

In addition to the roles of optical electrodes in transmitting and sensing light, optical electrodes can be configured to enable optical filtering, optical polarization, and/or the provision of a variable refractive index.

Optical electrodes can be controlled by a selectively transmitting light through the coupled optical fibers, such that individual optical electrodes transmitting light singly or in groups, or are activated sequentially, either singly or in groups. Such selective transmission also allows for directional illumination of a target tissue and sub-parts thereof. Additionally, lenses that serve to focus or diffract light, or optical filters that serve to select or block specific wavelengths, can be coupled to the optical fibers.

Other electrodes, manufactured of material detectable by magnetic resonance imaging (MRI), and which may not otherwise be electrically coupled, can be used to help detect the location of the catheter when being manipulated in an environment. These MRI electrodes can be located anywhere on the catheter, but can be very useful when included in the distal end.
Light guide adapters can be used to couple optical fibers to the optical electrodes. In the case of point and ring electrodes, light guide adapters can change the direction of light from predominantly longitudinal to predominantly transverse, and vice-versa using, for example, angled (cleaved or cut) surfaces for total internal reflection (analogous to a prism), and/or a reflective surface, such as a coated or polished surface. Light guide adapters can be formed integrally with optical fibers or optical electrodes, or can be included as non-integrated separate components.

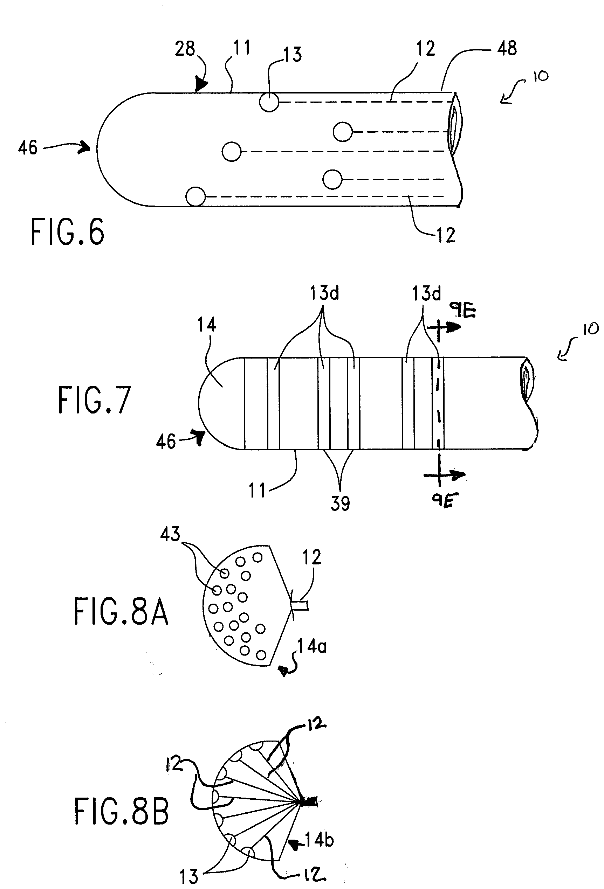

As shown in Figs. 5A and 6, the optical fibers 12 generally extend along the catheter shaft 28 to the proximal end 48 where the optical fibers 12 can be ultimately coupled to light sources and/or optical detecting devices and data processing devices. The optical fibers 12 can be predominantly configured to be aligned along the interior of the shaft 28, such that the fibers transmit light predominantly along the length of the catheter. Further, if desired, the optical fibers 12 can be bundled into cables.

In embodiment of an optical fiber-based catheter, optical fibers 12 transmit light to a target area via optical electrodes 13, and also transmit light, again via optical electrodes 13, to detecting and/or signaling processing devices. In embodiments, a single optical fiber can be used to both transmit and receive light, which can be accomplished by a coupler at or near the proximal end of the fiber. Alternatively, two (or even more) optical fibers can be used, wherein a first fiber transmits light to the target tissue, while a second fiber receives the signal produced by, for example, photo-activated substances. Light sources can comprise sources that transmit the required wavelengths (or can be filtered to do so) to excite the detectable substance loaded into or on the target tissue and are coupled to the optical fibers. For example, light sources can include light-emitting diodes (LEDs). The optical fibers can also be coupled at their proximal ends with devices for sensing a signal emitted from excited photodynamic detectable substances. The devices used for sensing can include, without limitation, an optical sensor, which can be, for example, photodiode arrays (PDAs) or charge coupled device (CCD) cameras. Optical filters can be coupled at the proximal (as well as distal) ends of the optical fibers to selectively pass and block desired wavelengths.

In embodiments, ANEP (aminonaphthylethenylpyridinium) dyes generally provide consistently sensitive probes for detection of submillisecond membrane potential changes. Di-4-ANEPPS (Invitrogen; Carlsbad, CA) has a fairly uniform 10% per 100 mV change in fluorescence intensity in a variety of tissue, cell and model membrane systems. ANEP dyes undergo changes in their electronic structure, and consequently their fluorescence spectra, in response to changes in the surrounding electric field. This optical response is sufficiently fast to detect transient potential changes in excitable cells, including single neurons, cardiac cells and intact tissue preparations. Furthermore, these dyes display a potential-dependent shift in their excitation spectra, thus permitting the quantifying of membrane potential using excitation ratio measurements. Other examples of ANEP dyes include di-8-ANEPPS, di-2-ANEPEQ, di-8-ANEPPQ, di-12-ANEPPQ, di-8-ANEPPQ, di-3-ANEPPDHQ, and di-4-ANEPPDHQ, all available from Invitrogen.

Some examples of other voltage sensitive dyes include JPW3067, JPW5034, and JPW5020, which have the same chromophore, but differ by the length of hydrocarbon (A. Matiukas et al. 2006. Am J Physiol Heart Circ Physiol 290: H2633-H2643). Other useful voltage-sensitive detectable substances include Voltage Sensor Probes (VSPs), which use Fluorescence Resonance Energy Transfer (FRET)-based voltage-sensing to measure changes in cellular membrane electrical potentials. Examples include CC2-DMPE, DiSBAC₂(3), and DiSBAC₄(3), also available from Invitrogen.

Fiber optic-based catheters can be used in various therapeutic and diagnostic applications, such as myocardial mapping and other similar applications and procedures. Accordingly, one of ordinary skill in the art will recognize and appreciate that the fiber optic-based devices and methods can be used in any number of therapeutic and diagnostic applications. Embodiments of the devices can be configured to conduct fluorescence signals from electrically active portions of the heart, to photosensitive elements and respective circuitry that convert the optical signal to a voltage signal. Once digitized, the signals can be visualized and analyzed with any number of techniques using a computer or processor. These signals can also be archived for inclusion in databases, such as disease or anatomical databases, or further analysis after the procedure.

Figs. 4A and 4B are partial side and cross sectional views, respectively, of an embodiment of a distal end of an optical fiber-based catheter 10 having a distal end 46 and proximal end 48. Fig. 4B shows a distal end of such a catheter 10 in cross-section as taken along dashed line 3-3 in Fig. 4A. Along the catheter shaft 28 are optical electrodes 13 arranged around the circumference of the catheter surface 11. Alternatively, optical electrodes can be distributed along one side of the catheter. The optical electrodes 13 can be flush, recess, or protrude from the surface 11 of the catheter. Metallic electrodes can also be included on the surface 11 of the catheter to aid in detection of the catheter when being used in a subject. Such electrodes can be electrically coupled or non-coupled. In this particular embodiment, the optical electrodes 13 are circular. However, such optical electrodes can, without limitation, also be diamond or rectangular in shape. In embodiments, the optical electrodes 13 can be flat, convex or concave at the surface of the catheter wall. The optical electrodes can be coupled to optical fibers 12. The optical fibers, which can be combined into a plurality to form cables, can be configured to serve at least two functions: (i) the fibers can transmit light from a light source to the body tissue; and, (ii) the fibers can transmit emitted/reflected light from the body tissue to the optical sensor. Each optical fiber or cable can be coupled to one or more optical sensors, such as a CCD camera or PDA device. CCD cameras, such as the Ixon camera manufactured by Andor of Ireland, can be designed for high speed and high resolution recording of fluorescence images. The output from the optical sensor can be provided or fed to a microprocessor, such as a personal computer, for image construction, visualization and/or other data analysis. In this manner, the action potential of a cluster of cells within the field of view of the optical fiber can be recorded. The action potential can contain information, such as the action potential duration, that conventional electrical recordings lack.

Figs. 5A-5C illustrate several embodiments of optical electrodes. As generally depicted in Fig. 5A, a partial side view of an embodiment of a distal end of an optical fiber-based catheter, one or more optical electrodes can have a wedge shape, 13a, a diamond shape 13b, or a rectangular shape 13c, as well as a rounded shape 13 (e.g., such as illustrated in Fig. 4B), or various combinations thereof. The optical electrodes 13 are distributed along the surface of the catheter shaft 28 at the distal end 46. The dashed lines generally represent optical fibers 12 coupled to the various examples of optical electrodes that extend to the proximal end of the catheter 48. The optical fibers 12 can be configured to extend through the catheter below the catheter surface 11 and can be coupled to signal detecting and/or processing components. Fig. 5B generally illustrates two alternative configurations of optical electrodes. As shown in the illustrated embodiment, a wedge-shaped optical electrode 13g can be coupled to an optical fiber 12 at a corner of the wedge disposed in the lumen 37 of the catheter. In Fig. 5C, a slightly different wedge-shaped optical electrode 13h can be coupled to an optical fiber 12 at a corner of the wedge displaced towards the distal end of the catheter.

Fig. 6 is a partial side view of a distal end of an embodiment of an optical fiber-based catheter that includes optical electrodes generally provided in a staggered or an off-set pattern or configuration. The catheter 10 includes a distal end 46, and optical fibers 12 coupled to optical electrodes 13 and extend towards the proximal end of the catheter 48. In the illustrated embodiment, the optical electrodes 13 are configured to be staggered, or off-set, along the surface 11 of the catheter shaft 28. Of course, more or less optical electrodes can be provided in various other patterns or configurations. Alternatively, optical electrodes can be distributed along one side (or just a portion of one side) of a catheter. With general reference to Figs. 4 and 6, the directionality of the signal and sensing can be achieved by controlling individual or groups of optical electrodes 13 by controlling coupled optical fibers 12, either individually or in groups. Alternatively, the optical electrodes 13 can be supplied only on a side, or localized portion of the catheter surface 11.

Fig. 7 is a partial side view of a distal end of an embodiment of an optical-based catheter 10 including ring optical electrodes 13d and a tip 14 optical electrode. The tip optical electrode 14 is provided at or about the distal end 46 of the catheter. The surface 39 (or at least the outward radially functional surface) of the ring optical electrodes 13d can be generally flush (as generally illustrated in the figure), recess, or protrude from the surface 11 of the catheter.

Figs. 8A and 8B generally illustrate, without limitation, two embodiments of tip optical electrodes 14a and 14b, respectively. Fig. 8A illustrates a tip optical electrode 14a wherein a single optical fiber 12 (or cable of a plurality of optical fibers) is coupled to the tip optical electrode 14a. The outer functional or "working" surface of the tip optical electrode 13e can be smooth, or have a textured surface, such as one that is "pixilated" with, for example, dimples, flat portions, etc. Such distal optical electrodes find special application in ablation procedures. Fig. 8B illustrates an embodiment of a tip optical electrode 14b including a plurality of optical electrodes 13. In the illustrated embodiment, each optical electrode 13 of the plurality of optical electrodes is individually coupled to an optical fiber 12. For embodiments in which a plurality of optical electrodes are provided on a tip, a filler or potting material can be included. Tip optical electrodes can also be polished or have reflective coatings applied.

As noted, ring optical electrodes 39 can have different shapes, and the edges that are exposed at the surface of the catheter can have different configurations. Figs. 9A-9C show embodiments of ring optical electrodes where the optical electrodes 13d are cone-shape and are coupled to optical fibers 12 at the tip of the cones. The circumference of the cones at the widest point are exposed at the surface of the catheter and can have flat 39a (Fig. 9A), concave 39b (Fig. 9B) or convex 39c (Fig. 9C) surfaces. Fig. 9D shows an alternative embodiment, wherein the ring optical electrode 13d has a narrow conical proximal end portion that can be flattened and directly coupled to the end surface of one or more optical fibers 12. Again, the protruding portion can be flattened, radially-rounded, and radially convexed, and can be partially coated or metallized. A bore or plurality of bores 41 for optical fibers can be formed off-center and the optical electrode so that more than one optical electrode can be operatively coupled to a singly light source. Fig. 9E is a cross-sectional view, taken along line 9E-9E of Fig. 7, showing an embodiment of a ring optical electrode having a cylindrical shape and a lumen. To create a direction signal, ring optical electrodes can be provided with a reflective coating around the circumference of the optical electrode, such that only a portion of the optical electrode is exposed and, as such, permits light to transmit from just a portion of the optical electrode. For example, ring optical electrodes can have a reflective coating provided around various extents of their periphery, for example, without limitation, about 200° to 350°, such as 200°, 225°, 250°, 275°, 300°, and 325°.

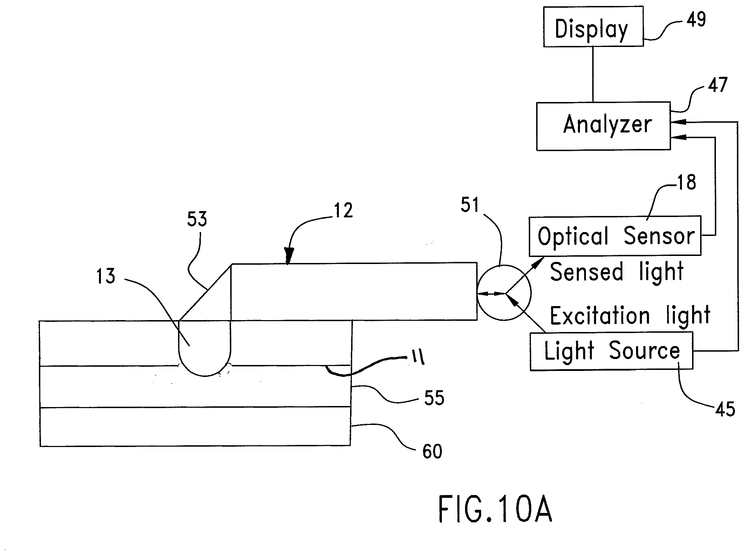

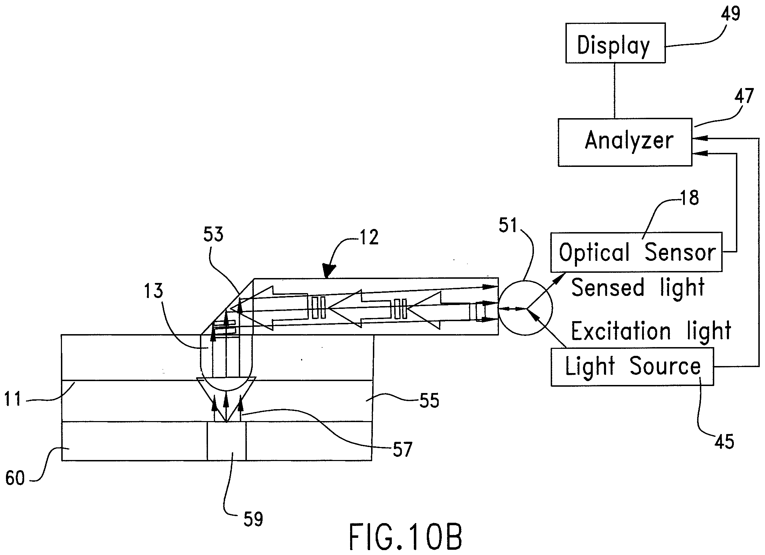

Fig. 10 is a representation that generally depicts the flow of light and signals associated with an embodiment of an optical fiber-based catheter in tissue provided with a voltage-sensitive detectable substance. As generally illustrated an optical fiber 12 can be coupled (e.g., via an optical coupler 51) at an optical sensor 18 and/or a light source 45 at or about a proximal end. The optical sensor 18 can be coupled to an analyzer 47, which in turn can additionally be coupled to a light source 45 and, if desired, can be separately coupled to a display 49. At the distal end of the optical fiber 12, a light guide adaptor 53 directs the optical fiber to an exterior surface of the catheter wall, which can be exposed to a fluid interface (such as blood or saline) to an optical electrode 13. The catheter can be placed adjacent to a target tissue, such as a portion of a myocardium 60. The target tissue can be provided or loaded with a voltage-sensitive dye. When the target tissue activates (e.g., depolarizes), the voltage sensitive dye can be activated by a suitable wavelength of light, which can be delivered through the optical electrode 13 by the optical fiber 12 coupled to the light source 45. The voltage sensitive dye can be activated, and light (block arrows) can be transmitted through the optical fiber 12, creating an optical response (generally designated 57), such as emitted light from the voltage sensitive dye. The optical fiber 12 can collect the signal and transmit the signal to the optical sensor 18.

While the preceding catheters are appropriate for endocardial and epicardial applications, as well as "whole chamber," local (conventional), and regional endocardial applications, basket array configurations, as discussed below, can be well suited for endocardial "whole chamber" applications. In one embodiment, the device includes a catheter that is similar in appearance, size, and maneuverability to a conventional basket assembly or array catheter, or a non-contact mapping catheter, and includes additional components that deliver and sense light at specific wavelengths. An embodiment of the catheter includes a catheter shaft with a proximal end and a distal end; a light source, which can comprise one or more light emitting diodes (LEDs); optical fibers or cables positioned inside the catheter shaft, where the optical fibers or cables extend from the proximal end of the catheter shaft to the distal end of the catheter shaft; an optical filter coupled to the optical fibers or cables; an optical sensor, such as a photodiode array (PDA) or charge-coupled device (CCD) camera; and an analyzer.

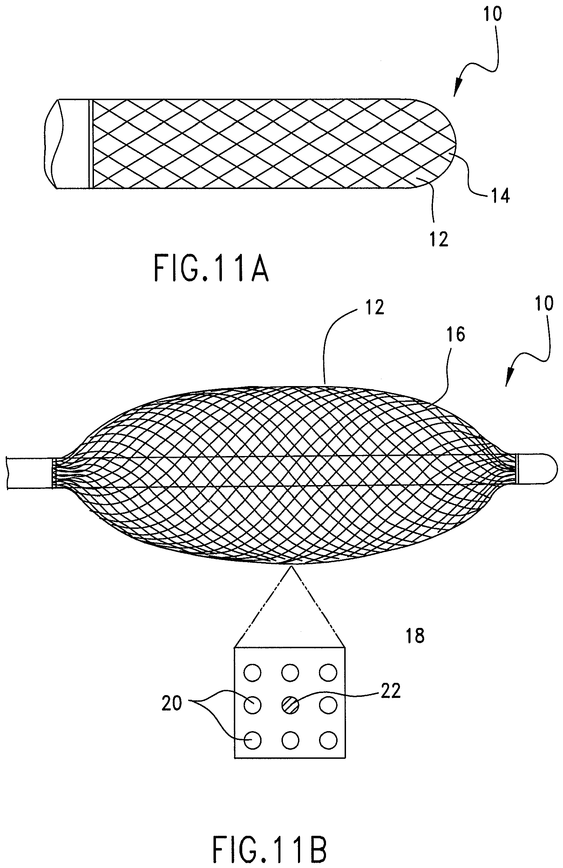

Figs. 11A and 11B depict side views of an embodiment of a fiber optic-based catheter 10 with optical fibers 12. Fig. 11A generally depicts the optical fibers 12 in an unexpanded position 14. Fig. 11B generally shows the optical fibers 12 in an expanded position 16, and generally represent an association with an optical sensor 18, such as a PDA device. The optical sensor or photodiode array device 18 comprises photodiodes 20 and a light emitting diode 22.

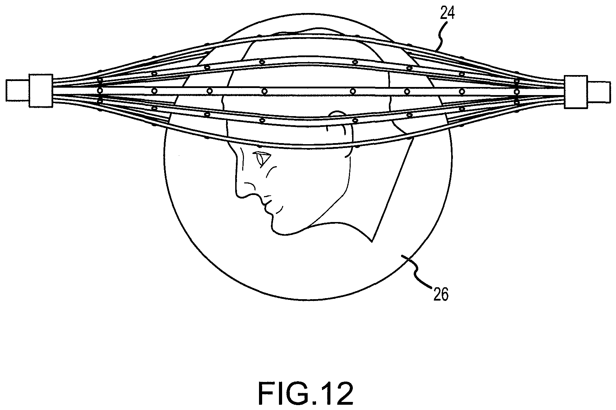

Fig. 12 generally illustrates an embodiment of a fiber optic-based steerable catheter including a plurality of splines 24 positioned about a dime coin 26. The splines 24 are shown next to the dime to provide a general comparison in relative size.

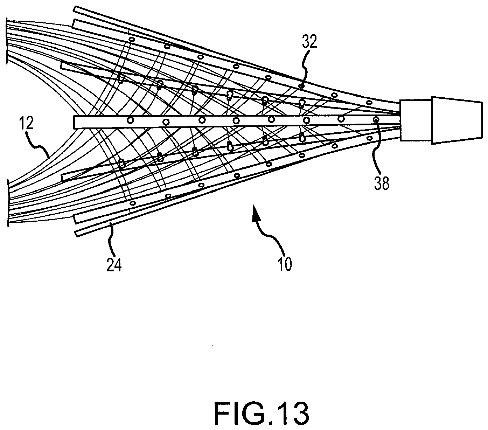

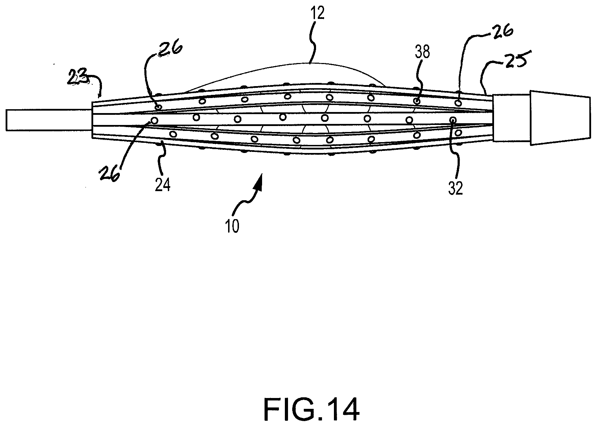

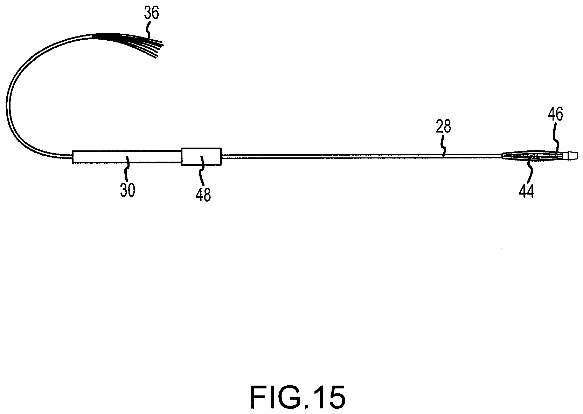

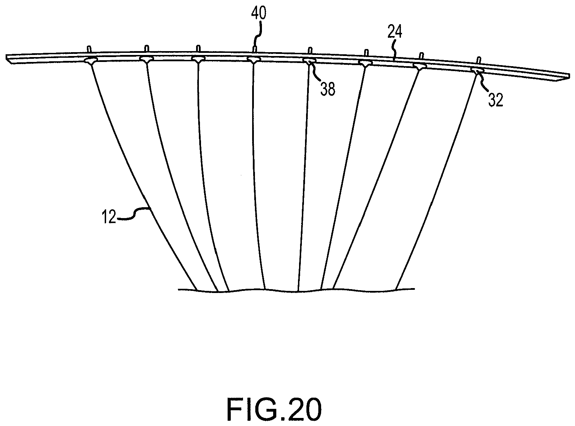

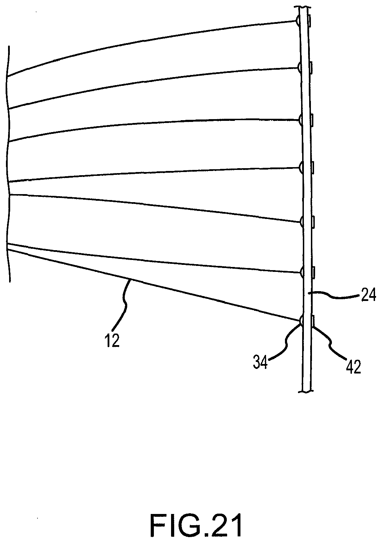

With reference to Figs. 13-18, an embodiment of a fiber optic-based catheter 10 is shown in various views. Fig. 13 shows a fiber optic-based catheter 10 having splines 24 coupled to or adapted for connection to a catheter shaft 28 (see Fig. 15) and handle portion 30 (see Fig. 15), and the splines are shown in an expanded position during construction. Fig. 14 shows the catheter of Fig. 13 showing splines 24 in an unexpanded position. The splines 24 can be made of a plastic material and can have a width of approximately 8 mm (millimeters) to 10 mm (millimeters), and a length of approximately 60 mm. The splines can be made of other suitable materials and of other suitable sizes. Fig. 13 shows a catheter with eight splines; however, the catheter can also have a single spline, or two-eight splines, or more than eight splines. Each spline 24 has a plurality of holes or openings 32 spaced along the length of each spline. Preferably, each spline has eight holes or openings. However, the spline can also have more than eight holes or less than eight holes. The holes or openings 32 can each have a diameter of approximately 300 micrometers in size, and each hole or opening can be spaced approximately 6 mm apart from another hole or opening, and the end holes can be spaced approximately 9 mm from the ends of the spline. However, the holes or openings can be of other suitably sized diameters and can be spaced at other suitable lengths apart from each other and from the ends of the spline. The optical fiber 12 is threaded through each hole or opening 32. Each optical fiber 12 can have a diameter of approximately 250 micrometers in size. However, the optical fibers can also have other suitably sized diameters. Cables of optical fibers can also be used. Preferably, the diameter of each optical fiber is smaller in size than the diameter of the corresponding hole or opening through which the optical fiber is threaded. Each optical fiber 12 has a connected end 34 (see Fig. 21) and a free end 36 (see Fig. 15). The connected end 34 is preferably connected to the corresponding hole in the spline. The optical fiber can be inserted through a hole and connected to the spline with an epoxy material 38 applied to each side of the hole in the spline. The epoxy material can be applied to the hole before insertion of the optical fiber and after insertion of the optical fiber. For example, the epoxy material is an epoxy encapsulant, such STYCAST® 2651 Black (Emerson & Cuming, Inc.; Billerica, Massachusetts). Any excess portion 40 (see Fig. 20) of an optical fiber that extends beyond the hole is preferably cleaved or cut off after the epoxy material has dried or set, so that the connected end of the optical fiber is flush with a side 42 of the spline (see Fig. 21). The free end 36 of the optical fiber can be connected to a photosensitive element for light to voltage conversion. In another embodiment, the catheter can comprise sixteen splines each with sixteen optical fibers, where each optical fiber has a diameter size of approximately 100 micrometers or smaller. This embodiment has a total of 256 light sensitive elements and four times the resolution of the embodiment having eight splines. Such an embodiment can include a total of 256 light sensitive elements and can, for instance, provide about four times the resolution of an embodiment having eight splines. With some embodiments, up to 3521, 250 µm optical fibers can be packaged within a 7 French catheter.

As shown in Fig. 15, the splines and optical fibers can be assembled into a basket assembly or array 44. Fig. 15 shows a fiber optic-based catheter 10 showing the basket assembly 44 at a distal end 46 of the catheter and the handle portion 30 at a proximal end 48 of the catheter. The free ends 38 of the optical fibers can be connected to a photosensitive element such as a photodetector or optical sensor during operation. The catheter can have a conventional cylindrical shape for insertion into a body. Once the catheter is positioned inside the body at the target location, such as the interior of the heart, the catheter can be expanded so that the basket assembly at the distal portion of the catheter expand into an elliptical shape or shell.

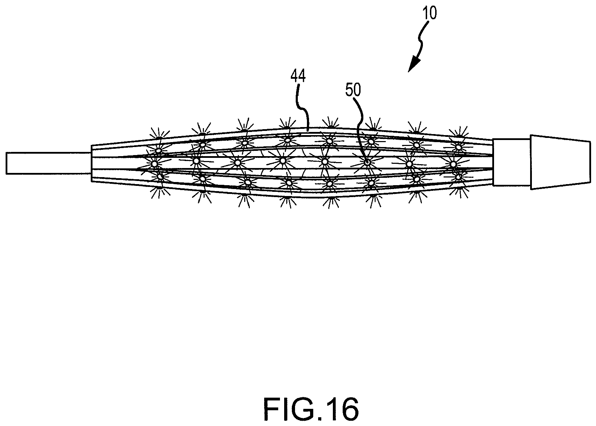

Fig. 16 shows an embodiment of a fiber optic-based catheter 10 showing light conduction of the basket assembly 44 in an unexpanded position. A plurality of points of light 50 is shown. The points of light are emitted from a light source. Preferably, the light source comprises one or more LEDs. However, other suitable light sources can also be used. The selected light source emits light at a peak wavelength which activates a selected photodynamic drug or detectable substance. Each light source can be coupled to an optical fiber or cable that extends the length of the catheter shaft to transmit light through strands or cables distributed within the basket assembly to illuminate the body tissue. The points of light activate the photodynamic agent in the target tissue, during which time the activated photodynamic detectable substance emits or reflects light at a wavelength different from the activation light. The emitted/reflected light can be collected in an assembly or arrays of optical fibers or cables at multiple points in the larger basket assembly. The optical fibers or cables can be coupled to optical filters, at either the distal end or the proximal end.

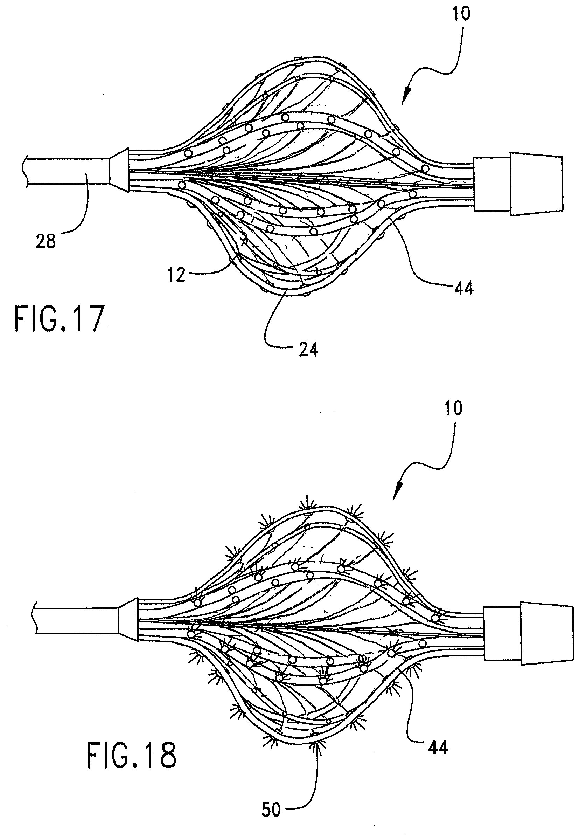

Fig. 17 shows an embodiment of a fiber optic-based catheter 10 showing the basket assembly 44 in an expanded position and the catheter shaft 28. Fig. 18 shows an embodiment of a fiber optic-based catheter 10 showing light conduction of the basket assembly 44 in an expanded position. A plurality of points of light 50 is shown. The points of light are emitted from a light source, such as from one or more LEDs. Light may be advantageously delivered orthogonal to the cleaved surface of each optical fiber or cable.

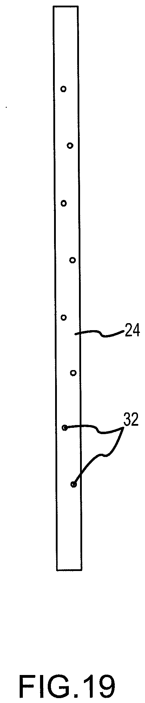

Fig. 19 shows an embodiment of a spline 24 of a fiber optic-based catheter. Figs. 20-23 show an fiber-optic based catheter in some steps of construction. Fig. 20 shows a fiber optic based catheter wherein optical fibers 12 are inserted into holes 32 in the spline 24 of the fiber optic-based catheter. Epoxy material 38 can be applied to each side of each hole in the spline, preferably before and after insertion of the fiber into the hole. Fig. 21 shows cleaved or cut optical fibers 12 that have been inserted into and adhered to the spline 24 of the fiber optic-based catheter. The excess portion 40 (see Fig. 20) of the optical fiber that extends beyond the hole is cleaved or cut off after the epoxy material has dried or set, so that the connected end of the optical fiber is flush with the side 42 of the spline (see Fig. 21). The optical fiber cut end can then be polished.

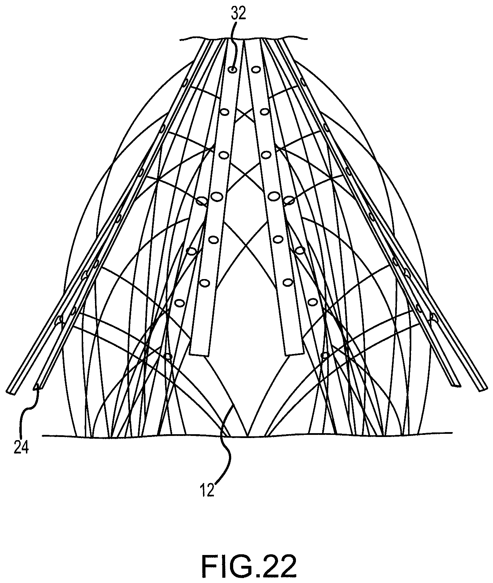

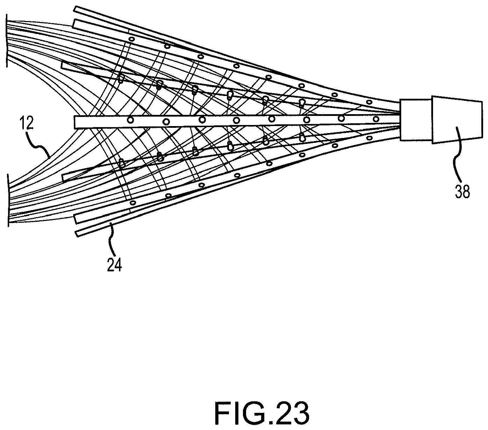

Fig. 22 shows an assembly of optical fibers 12 and splines 24 of the fiber optic-based catheter. Preferably, the optical fibers are woven together before assembling the basket assembly 44. Fig. 23 shows the fiber optic-based catheter showing an outer lumen 52. In the case of multiple outer lumens, the splines can be inserted circumferentially between two outer lumens. Each lumen can comprise a fluorinated ethylene propylene heat shrink lumen. However, lumens made of other suitable materials can also be used. In one embodiment, the outer lumens can be made by using an approximately 0.3 inch diameter fluorinated ethylene propylene heat shrink tubing material, cutting two pieces of the tubing material each approximately one inch long, recovering an approximately 0.2 inch diameter piece of the tubing material, inserting an approximately 0.198 inch gage pin into the piece of the tubing material, applying heat to a desired portion of the tubing material, cleaving or cutting a desired portion of the tubing material beyond the neck portion, inserting the splines circumferentially between the two outer lumens of tubing material, applying epoxy material between the splines, shifting the splines so the epoxy adheres, allowing the epoxy to dry, and if necessary, applying additional epoxy to the outer diameter of the lumen or catheter.

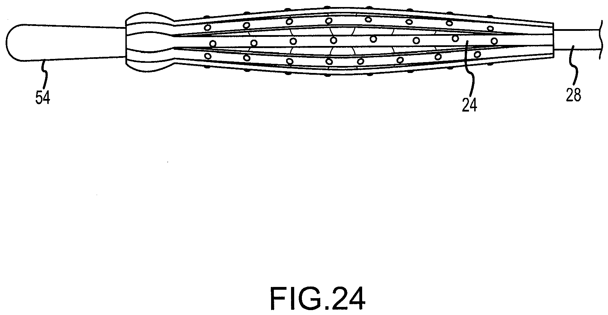

Fig. 24 shows the fiber optic-based steerable catheter showing pull wires 54 at the distal end 46 of the catheter. The pull wires can be connected to an actuator element that is coupled to the catheter. When assembling the pull wires to the handle, an 11 Fr (French) AGILIS introducer (AGILIS is a registered trademark of St. Jude Medical, Atrial Fibrillation Division, Inc. of Minnetonka, Minnesota) can be used, and the handle can be removed by unscrewing the actuator and pulling the handle toward the distal end of the catheter. The handle can be advantageously adapted for connecting the actuation element or elements so that a user can selectively manipulate the distal end of the catheter assembly to deflect in one or more directions (e.g., up, down, left, and right). The handle is operative to effect movement (i.e., deflection) of the distal end of the catheter assembly. The catheter can be modified so that a portion of the lumen remains beyond the handle. The optical fibers are advantageously inserted through the lumen from the proximal end to the distal end. The pull wires can be inserted through the inner diameter of the proximal end and the distal end of the basket assembly. Pull wires are not interwoven with the optical fibers in the basket assembly. The pull wires can be bent inwardly, and a stainless steel solder can be applied to create U-shaped pull wires. If an inner, proximal lumen overhangs the handle, it can be removed or cut off so that any applied epoxy is flush with the lumen.

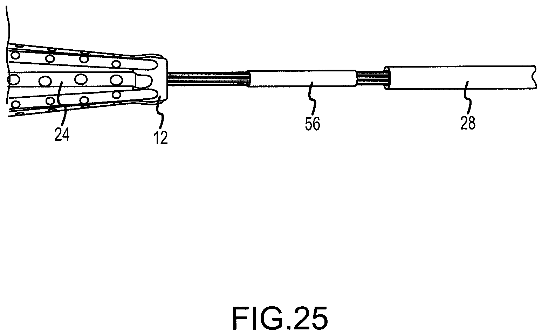

Fig. 25 shows optical fibers 12 of the fiber optic-based catheter wrapped with tape 56 for protecting the optical fibers when adhering the lumen to the catheter and handle. When assembling the basket assembly to the catheter and handle, tape, such as polytetrafluoroethylene (PTFE) tape, can be wrapped around the optical fibers to protect the optical fibers. The surface of the distal end of the catheter lumen can be modified such as by rubbing it with sandpaper. The basket assembly can then be attached to the outer lumen of the catheter with ultraviolet (UV) light application and allowed to dry. Pull wires can then be adhered with epoxy to the inner diameter of the distal tip of the basket. Fig. 26 shows the fiber optic-based catheter showing pull wires 54 in a distal tip 58.

The fiber optic-based catheters can also be used to acquire fluorescence signals at a rapid rate to distinguish key features of each action potential. Alternatively, the catheter can be used with a long exposure time to monitor average fluorescence. Diseased tissue is often associated with depressed voltage amplitudes, and current conventional or non-contact mapping electrodes are used to identify these low voltage regions. Depressed voltage amplitudes typically correspond to low averaged fluorescence signals over the long exposure time.

The optical fibers or cables of the catheter can also be used to deliver light for photodynamic-based therapy. By administering drugs that induce cell necrosis or apoptosis upon exposure to light of a particular frequency, particular regions of the heart can be optically ablated. For example, introducing the basket assembly into the ostium of a pulmonary vein, a frequent target for ablation of atrial fibrillation, and applying the excitation light only through a particular fiber on each spline, a circumferential lesion can be photodynamically created with a single light application. Similar lesions can be created with conventional radio frequency (RF) ablation by sequentially placing the catheter tip at spots around the ostium and applying energy for some duration at each point. In some cases, the amount of drug that is delivered can be controlled by the strength and proximity of illumination, as well as time of illumination.

Mapping tissue inside a body cavity can be accomplished with fiber optic-based catheters as described above. In one embodiment, the method is directed to photodynamic-based myocardial mapping. The method comprises the step of providing a fiber optic-based catheter assembly. The method further comprises the step of inserting the catheter assembly into the body cavity. The method further comprises the step of actuating the distal end of the catheter against or next to a target tissue. Fluorescence signals from the target tissue of an intact human heart are transmitted to photosensitive elements and respective circuitry that convert the optical signal to a voltage signal. The method further comprises the step of once the signals are digitized, visualizing the signals and analyzing the signals with any number of techniques using a digital computer. The method can further comprise the step of archiving the signals for inclusion in databases, such as disease or anatomical databases, or for further analysis after the procedure.

Specifically, in a first step, the target tissue is perfused or loaded with a photodynamic detectable substance, such as a, voltage-sensitive dye. The substance is relatively inert until activated by radiation of a specific wavelength. Upon activation, the substance emits light of a specific wavelength when excited. Examples of detectable substances include electrochromic and potentiometric dyes, such as di-2-ANEPEQ, di-4-ANEPPS, or di-8-ANEPPS.

The substance can be introduced into the tissue in a variety of ways such that the substance is absorbed into the cells in the tissue or binds to cell membranes. For example, the substance can be introduced through in situ delivery, arterial delivery and/or systemic delivery. One method of in-situ delivery is electroporation, in which a site-limited electric shock is used to create an electric field to cause expansion of the cells in the tissue for a period of time to allow the substance to penetrate cell membranes, thus entering the cells. Alternative methods of in situ delivery can be applying an electrical field on the substance itself or using acoustic waves (e.g. ultrasound) to break through the tissue boundary. Alternatively, the substance can be infused through the artery, such as the coronary artery, to allow perfusion into the tissue. It should be understood that these methods of introducing the substance to the tissue are exemplary.

In a next step, light at an excitation wavelength is applied to the target tissue. For example, this can be accomplished by the fiber optic-based catheter, wherein a light source is coupled to an optical coupler that is coupled to an optical fiber. The light can be filtered to apply the appropriate wavelength to excite the loaded, detectable photodynamic substance. The optical fiber is coupled to an optical electrode that is at the surface of the catheter and adjacent to the target tissue.

Once the excitation wavelength has been applied, the photodynamic voltage sensitive dye emits a signal that can be sensed by the optical fiber-based catheter. This signal can be sensed using the same optical fiber or cable that delivered the excitation wavelength, or by a separate optical fiber or cable. The signal travels through an optical electrode by an optical fiber or cable, through a coupled optical coupler and to a coupled optical sensor. The sensed signal can also pass through filters to select for the sensed signal and to reduce background noise.

From the optical sensor, the signal is translated into a transmembrane potential, and such information can be displayed on a computer screen as an image. The ENSITE® system (Saint Jude Medical; St. Paul, MN) provides exemplary capabilities, although this is an electrical based system.

Fiber optic-based catheters, as described above, can be used for delivering light for photodynamic-based therapy to a target tissue, such as myocardial tissue. Fiber optic-based catheters can also be used to acquire fluorescence signals at a rapid rate to distinguish key features of each action potential.

Although a number of representative embodiments according to the present teachings have been described above with a certain degree of particularity, those skilled in the art could make numerous alterations to the disclosed embodiments without departing from the scope of this invention. For example, different types of catheters can be manufactured or result from the inventive process described in detail above. For instance, catheters used for diagnostic purposes and catheters used for therapeutic purposes can both be manufactured using the inventive process. Additionally, all directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use. Joinder references (e.g., attached, coupled, connected, and the like) are to be construed broadly and can include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure can be made without departing from the invention.

## Claims

1. A photodynamic tissue mapping device (10), comprising:
a shaft (28) with a proximal end and a distal end (46);
at least one optical electrode (13, 14, 39) at the distal end (46) of the shaft (28);
at least one optical fiber (12) positioned inside the shaft (28), wherein the at least one optical fiber (12) extends from the distal end (46) of the shaft (28), coupled to the at least one optical electrode (13) at an outer surface of the shaft (28), to the proximal end (48) of the shaft (28);
a light source (45) coupled to the at least one optical fiber (12) at a proximal end of the at least one optical fiber (12);
an optical sensor (18) coupled to an at least one of the optical fibers or other receiving optical fiber; and
an analyzer (47) coupled to the optical sensor (18);
the optical sensor (18) is adapted to receive signals emitted by a voltage-sensitive dye and indicative of an activation of tissue in response to exposure of the voltage-sensitive dye to the light source provided via the at least one optical fiber, wherein detecting the voltage-sensitive detectable substance is mapping the electrical activity in the target portion of heart, and
the at least one optical electrode (13, 14, 39) is a plurality of optical electrodes and the at least one optical fiber (12) is a plurality of optical fibers, wherein the plurality of optical electrodes (13, 14, 39) is distributed along the shaft.

2. The device (10) of claim 1, further comprising an optical filter coupled to the at least one optical fiber.

3. The device (10) of claim 1, further comprising a light guide adapter that couples the at least one optical fiber (12) to the at least one optical electrode (13, 14, 39).

4. The device (10) of claim 1, wherein the light source (45) comprises at least one light emitting diode (LED), and wherein the optical sensor (18) comprises a photodiode array (PDA) or charge coupled device (CCD) camera.

5. The device (10) of claim 1, wherein the device is a catheter selected from the group consisting of a basket assembly, array, and non-contact mapping catheter.

6. The device (10) of claim 1, wherein the plurality of optical electrodes (13, 14, 39) is preferably distributed on a side of the shaft (28).

7. The device (10) of claim 1, wherein the at least one optical electrode (13, 14, 39) is selected from the group consisting of a point electrode, a ring electrode, and a tip electrode.

8. The device (10) of claim 1, wherein an outer surface of the at least one optical electrode (13, 14, 39) is convex, planar, or concave.

9. The device (10) of claim 1, wherein the at least one optical electrode (13, 14, 39) comprises glass, a solid comprising polymers, a low durometer polymer, or is encapsulated with a fluid or gel.

10. The device (10) of claim 1, wherein the at least one optical electrode (13, 14, 39) enables optical filtering, optical polarization or variable refractive index.

11. The device (10) of claim 5, wherein the catheter is a basket assembly catheter, further comprising:
at least one spline (24) having a least one opening (32), the at least one spline (24) attached to a distal end of a catheter shaft (28); and
a handle portion (30),
wherein the at least one optical fiber (12) is connected to, or threaded through the at least one opening (32) in the at least one spline (24),
wherein the device (10) preferably comprises a plurality of splines (24), the plurality of splines (24) preferably comprising 8 splines or 16 splines.

12. The device (10) of claim 11, wherein the at least one spline (24) comprises 8 openings (32).

13. The device (10) of claim 11, wherein an end of the at least one optical fiber (12) is flush with a surface of the at least one spline (24).

14. The device (10) of claim 11, wherein the catheter comprises at least one outer lumen or at least one inner lumen (52), wherein the plurality of optical fibers (12) is threaded through the at least one outer or at least one inner lumen (52).

15. The device (10) of claim 1, further comprising at least one pull wire (54) connected at a distal end of the device and coupled to an actuator element at a posterior end of the device.

## Patentansprüche

1. Photodynamische Gewebeabbildungsvorrichtung (10), mit
einem Schaft (28) mit einem proximalen Ende und einem distalen Ende (46), zumindest einer optischen Elektrode (13, 14, 39) an dem distalen Ende (46) des Schaftes (28),
zumindest einer optischen Faser (12), die im Inneren des Schaftes (28) positioniert ist, bei der sich die zumindest eine optische Faser (12) von dem distalen Ende (46) des Schaftes (28) und mit der zumindest einen optischen Elektrode (13) an einer äußeren Oberfläche des Schaftes (28) gekoppelt zu dem proximalen Ende (48) des Schaftes (28) erstreckt,
einer Lichtquelle (46), die mit der zumindest optischen Faser (12) an einem proximalen Ende der zumindest einen optischen Faser (12) gekoppelt ist,
einem optischen Sensor (18), der mit der zumindest einen der optischen Faser oder einer empfangenden optischen Faser gekoppelt ist, und
einer Analyseeinheit (47), die mit dem optischen Sensor (18) gekoppelt ist,
bei der der optische Sensor (18) dazu angepasst ist, dass er Signale empfängt, die von einem spannungssensitiven Färbemittel emittiert werden und eine Aktivierung von Gewebe als Reaktion auf die Belichtung des spannungssensitiven Färbemittels mit der über die zumindest eine optische Faser bereitgestellten Lichtquelle anzeigen, bei der die Erfassung der spannungssensitiven erfassbaren Substanz die elektrische Aktivität in einem Zielbereich des Herzens abbildet, und
die zumindest eine optische Elektrode (13, 14, 39) eine Mehrzahl von optischen Elektroden ist und die optische Faser (12) eine Mehrzahl von optischen Fasern ist, bei der die Mehrzahl der optischen Elektroden (13, 14, 39) entlang des Schaftes verteilt ist.

2. Vorrichtung (10) nach Anspruch 1, die weiter einen optischen Filter aufweist, der mit der zumindest einen optischen Faser gekoppelt ist.

3. Vorrichtung (10) nach Anspruch 1, die ferner einen Lichtführungsadapter aufweist, der die zumindest eine optische Faser (12) mit der zumindest einen optischen Elektrode (13, 14, 39) koppelt.

4. Vorrichtung (10) nach Anspruch 1, bei der die Lichtquelle (45) zumindest eine lichtemittierende Diode (LED) aufweist, und bei der der optische Sensor (18) eine Photodiodenanordnung (PDA) oder eine ladungsgekoppelte Vorrichtungs-(CCD-)Kamera aufweist.

5. Vorrichtung (10) nach Anspruch 1, bei der die Vorrichtung ein Katheter ist, der aus der Gruppe gewählt ist, die aus einer Korbbaugruppe, einer Reihenanordnung und einem Nichtkontaktabbildungskatheter besteht.

6. Vorrichtung (10) nach Anspruch 1, bei dem die Mehrzahl der optischen Elektroden (13, 14, 39) bevorzugt auf einer Seite des Schaftes (28) verteilt ist.

7. Vorrichtung (10) nach Anspruch 1, bei der die zumindest eine optische Elektrode (13, 14, 39) gewählt ist aus der Gruppe, die aus einer Punktelektrode, einer Ringelektrode und einer Spitzenelektrode besteht.

8. Vorrichtung (10) nach Anspruch 1, bei der eine äußere Oberfläche der zumindest einen optischen Elektrode (13, 14, 39) konvex, planar oder konkav ist.

9. Vorrichtung (10) nach Anspruch 1, bei der die zumindest eine optische Elektrode (13, 14, 39) Glas, einen Feststoff, der Polymere aufweist, ein Polymer mit niedrigem Durometer aufweist oder mit einer Flüssigkeit oder einem Gel eingekapselt ist.

10. Vorrichtung (10) nach Anspruch 1, bei der die zumindest eine optische Elektrode (13, 14, 39) optisches Filtern, optische Polarisation oder einen variablen Berechnungsindex ermöglicht.

11. Vorrichtung (10) nach Anspruch 5, bei dem der Katheter ein Korbbaugruppenkatheter ist, der ferner
zumindest einen Spline (24), der zumindest eine Öffnung (32) aufweist, wobei der zumindest eine Spline (24) an ein distales Ende eines Katheterschaftes (28) angebracht ist, und
einen Handgriffbereich (30) aufweist,
bei der die zumindest eine optische Faser (12) mit der zumindest einen Öffnung (32) bei dem zumindest einen Spline (24) verbunden ist oder durch diese gefädelt ist,
bei der die Vorrichtung (10) bevorzugt eine Mehrzahl von Splinen (24) aufweist, bei der die Mehrzahl von Splinen (24) bevorzugt 8 Spline oder 16 Spline aufweist.

12. Vorrichtung (10) nach Anspruch 11, bei der der zumindest eine Spline (24) 8 Öffnungen (32) aufweist.

13. Vorrichtung (10) nach Anspruch 11, bei der ein Ende der zumindest einen optischen Faser (12) bündig mit einer Oberfläche des zumindest einen Splines (24) ist.

14. Vorrichtung (10) nach Anspruch 11, bei der der Katheter zumindest ein äußeres Lumen oder zumindest ein inneres Lumen (52) aufweist, bei der die Mehrzahl von optischen Fasern (12) durch das zumindest eine äußere oder das zumindest eine innere Lumen (52) gefädelt ist.

15. Vorrichtung (10) nach Anspruch 1, die ferner zumindest einen Ziehdraht (54) aufweist, der mit einem distalen Ende der Vorrichtung verbunden ist und mit einem Betätigungselement an einem hinteren Ende der Vorrichtung gekoppelt ist.

## Revendications

1. Dispositif de mise en correspondance de tissu photodynamique (10), comprenant :
un arbre (28) avec une extrémité proximale et une extrémité distale (46) ;
au moins une électrode optique (13, 14, 39) à l'extrémité distale (46) de l'arbre (28) ;
au moins une fibre optique (12) positionnée à l'intérieur de l'arbre (28), dans lequel ladite au moins une fibre optique (12) s'étend de l'extrémité distale (46) de l'arbre (28), couplée à ladite au moins une électrode optique (13) au niveau d'une surface extérieure de l'arbre (28), à l'extrémité proximale (48) de l'arbre (28) ;
une source de lumière (45) couplée à ladite au moins une fibre optique (12) à une extrémité proximale de ladite au moins une fibre optique (12) ;
un capteur optique (18) couplé à au moins une fibre parmi les fibres optiques ou une autre fibre optique de réception ; et
un analyseur (47) couplé au capteur optique (18) ;
le capteur optique (18) est adapté pour recevoir des signaux émis par un colorant sensible à la tension et indiquant une activation de tissu en réponse à l'exposition du colorant sensible à la tension à la source de lumière fournie via ladite au moins une fibre optique, dans lequel la détection de la substance détectable sensible à la tension met en correspondance l'activité électrique dans la partie cible du cœur,
et
ladite au moins une électrode optique (13, 14, 39) est une pluralité d'électrodes optiques et ladite au moins une fibre optique (12) est une pluralité de fibres optiques, dans lequel la pluralité d'électrodes optiques (13, 14, 39) est répartie le long de l'arbre.

2. Dispositif (10) selon la revendication 1, comprenant en outre un filtre optique couplé à ladite au moins une fibre optique.

3. Dispositif (10) selon la revendication 1, comprenant en outre un adaptateur de guide de lumière qui couple ladite au moins une fibre optique (12) à ladite au moins une électrode optique (13, 14, 39).

4. Dispositif (10) selon la revendication 1, dans lequel la source de lumière (45) comprend au moins une diode électroluminescente (DEL), et dans lequel le capteur optique (18) comprend un réseau de photodiodes (PDA) ou une caméra à dispositif de couplage de charge (CCD).

5. Dispositif (10) selon la revendication 1, dans lequel le dispositif est un cathéter choisi dans le groupe constitué d'un ensemble de panier, un réseau et un cathéter de mise en correspondance sans contact.

6. Dispositif (10) selon la revendication 1, dans lequel la pluralité d'électrodes optiques (13, 14, 39) est de préférence répartie sur un côté de l'arbre (28).

7. Dispositif (10) selon la revendication 1, dans lequel ladite au moins une électrode optique (13, 14, 39) est choisie dans le groupe constitué d'une électrode à pointe, une électrode annulaire et une électrode d'extrémité.

8. Dispositif (10) selon la revendication 1, dans lequel une surface extérieure de ladite au moins une électrode optique (13, 14, 39) est convexe, plane ou concave.

9. Dispositif (10) selon la revendication 1, dans lequel ladite au moins une électrode optique (13, 14, 39) comprend du verre, un solide comprenant des polymères, un polymère à faible durométrie, ou est encapsulée avec un fluide ou un gel.

10. Dispositif (10) selon la revendication 1, dans lequel ladite au moins une électrode optique (13, 14, 39) permet un filtrage optique, une polarisation optique ou un indice de réfraction variable.

11. Dispositif (10) selon la revendication 5, dans lequel le cathéter est un cathéter d'ensemble de panier, comprenant en outre :
au moins une cannelure (24) ayant au moins une ouverture (32), ladite au moins une cannelure (24) étant fixée à une extrémité distale d'un arbre de cathéter (28) ; et
une partie de poignée (30),
dans lequel ladite au moins une fibre optique (12) est connectée à, ou enfilée à travers ladite au moins une ouverture (32) dans ladite au moins une cannelure (24),
dans lequel le dispositif (10) comprend de préférence une pluralité de cannelures (24), la pluralité de cannelures (24) comprenant de préférence 8 ou 16 cannelures.

12. Dispositif (10) selon la revendication 11, dans lequel ladite au moins une cannelure (24) comprend 8 ouvertures (32).

13. Dispositif (10) selon la revendication 11, dans lequel une extrémité de ladite au moins une fibre optique (12) est au ras d'une surface de ladite au moins une cannelure (24).

14. Dispositif (10) selon la revendication 11, dans lequel le cathéter comprend au moins une lumière extérieure ou au moins une lumière intérieure (52), dans lequel la pluralité de fibres optiques (12) est enfilée à travers ladite au moins une lumière extérieure ou au moins une lumière intérieure (52).

15. Dispositif (10) selon la revendication 1, comprenant en outre au moins un fil de traction (54) connecté à une extrémité distale du dispositif et couplé à un élément d'actionnement à une extrémité postérieure du dispositif.
